# EUROPEAN PATENT SPECIFICATION

(11) **EP 2 049 182 B1**
(45) Date of publication and mention of the grant of the patent: **26.11.2014**
(21) Application number: 07836458.5
(22) Date of filing: 02.08.2007
(51) Int. Cl.: A61M 25/00, A61M 25/10

(54) **METHOD OF MICROPLEATING A CATHETER BALLOON WITH MULTIPLE MICROPLEATS**
HERSTELLUNGSVERFAHREN FÜR EINEN BALLONKATHETER MIT MEHREREN MIKROFALTEN
PROCÉDÉ DE FABRICATION D'UN BALLON DE CATHÉTER COMPRENANT DES MICROPLIS MULTIPLES

(30) Priority: 07.08.2006 US 500793
(43) Date of publication of application: 22.04.2009
(73) Proprietor: Gore Enterprise Holdings, Inc., Newark, DE 19714-9206 (US)
(72) Inventor: ESKAROS, Sherif, Elkton, MD 21921 (US); HOUGHTON, Michael, Newark, DE 19711 (US); NEWCOMB, Kenneth, Wilmington, DE 19803 (US); ROEBER, Peter, J., Wallingford, PA 19086 (US); TOWLER, Jeffrey, Wilmington, DE 19803 (US)
(74) Representative: Shanks, Andrew
(86) International application number: PCT/US2007/017307
(87) International publication number: WO 2008/021019

(56) References cited:
- EP-A- 0 737 488
- US-A- 5 308 356
- US-A- 5 893 840
- US-A- 6 010 480
- US-A- 6 013 092
- US-A1- 2002 163 104
- US-A1- 2003 130 716

## Description

### BACKGROUND OF THE INVENTION

Catheter balloon designs have incorporated various methods with the intent of attaining a low catheter profile. A low catheter profile is desirable because it reduces the likelihood of catheter based complications. In addition, lower profiles allow use of the catheter in tortuous paths and therefore enable access to more parts of an anatomy, and allow use of smaller guide catheters. The previous approach to minimizing catheter profiles involves the use of materials which allow formation of thinner balloon walls which are subsequently folded and packed to achieve lower profiles. While such designs achieve relatively low catheter profiles, they fail to produce designs conducive to uniform stent deployment.

Those balloon designs that have achieved greater uniformity in stent deployment generally exhibit multiple folds, also known as "wings" or "pleats". Packing designs known in the art include tri-folded balloon profiles and other multiple canted wing profiles. These packing designs are not known to achieve a uniform expansion when used in stenosis dilation procedures, thus increasing the likelihood of contributing to arterial trauma. Also, non-uniform stent deployment leads to non-uniform stent cell size per area, which may lead to tissue and/or plaque prolapse, which in turn results in a smaller luminal area for blood flow.

US 6,013,092 relates to methods for folding a catheter mounted balloon. The methods generally comprise folding a balloon portion that is in a flattened configuration at least twice, in opposite directions, so that any rotational forces created by the unfolding of the balloon as it is inflated will counteract each other. Catheter balloons having longitudinal furrows; and catheters and systems for implanting endoluminal devices with such balloons are also disclosed.

US 2002/0163104 relates to an apparatus for folding a catheter balloon in two steps. In the first step the balloon is folded into a shape with curved wings and angled wing bases (a spiral-pleated shape) and in the second step the balloon is folded tightly round the shaft by means of radial compression through a diameter reduction.

US 5,308,356 relates to a passive perfusion angioplasty catheter which defines at least one passage between a surface of the balloon and the interior wall of an artery to permit blood flow therethrough when the balloon member is pressed against the artery wall. Examples of pleated balloon members, which provide the passage for blood flow are described and illustrated.

US 2003/0131716 relates to a non-compliant balloon with compliant top layer to protect coated stents during expansion. In the uninflated/deflated state the balloon comprises longitudinal wings, which wrap around the longitudinal axis of the non-compliant balloon.

EP 0 737 488 relates to a balloon catheter with a lobated balloon. The expandable balloon member comprises a number of relatively stiff sections extending on a small diameter in a longitudinal direction of the basic body and relatively pliable sections extending in between wherein the relatively pliable sections form lobes in an expanded state of the balloon member.

US 5,893,840 relates to a balloon catheter which comprises microcapsules of a drug on its surface. In some embodiments, these microcapsules are held in place mechanically by folds in the balloon. As the balloon inflates, the folds are eliminated which expels the microcapsules into the treatment site.

(US 6,010,480) relates to an expansible balloon cathether which may be compacted by pleating about a central tube. As the balloon is expanded, the pleats open into a generally circular configuration.

See as well US 5 147 302 and WO 9511718.

What is needed is a flexible catheter balloon packing design which facilitates radial symmetry about the circumference of the balloon during inflation in vivo so as to provide a smaller introducer sheath so as to reduce potential trauma to the access site during introduction and to the arterial walls during inflation or device deployment. Prevention of plaque prolapse through stent cells post deployment is also desired. The present invention solves this long felt need and contributes to uniform stent deployment.

### SUMMARY OF THE INVENTION

The present invention provides a method of forming the micropleats on a balloon according to claim 1.

The examples provides a catheter balloon formed of at least one balloon material having a longitudinal axis with micropleats distributed about the circumference of the balloon resulting in a low profile and an essential symmetry upon inflation. The balloon of the examples is able to achieve a low profile due to smaller introducer sheath requirements. Additionally, the balloon reduces vessel trauma through radial concentric inflation of the balloon itself.

### DESCRIPTION OF THE DRAWINGS

Figure 1 shows a cross section of an uninflated micropleated balloon.
Figure 2 shows a cross section of an uninflated folded balloon which embodies features of the prior art.
Figure 3 shows an uninflated micropleated catheter balloon.
Figure 4 shows a side view of an inflated micropleated catheter balloon.
Figure 5 shows a top view of a deflated micropleated catheter balloon.
Figure 6 shows a schematic of a micropleated balloon affixed to a catheter shaft.
Figure 7 shows a deflated elastomer cover on a non-inflated balloon which is not yet micropleated.
Figure 8 shows a deflated elastomer tube covering an inflated balloon which is not yet micropleated.

### DETAILED DESCRIPTION OF THE INVENTION

There are described examples of providing a catheter balloon with the balloon material formed into micropleats distributed about the circumference of the balloon. The micropleats reduce the entry profile of the balloon and are formed to pull taut in an inflated state.

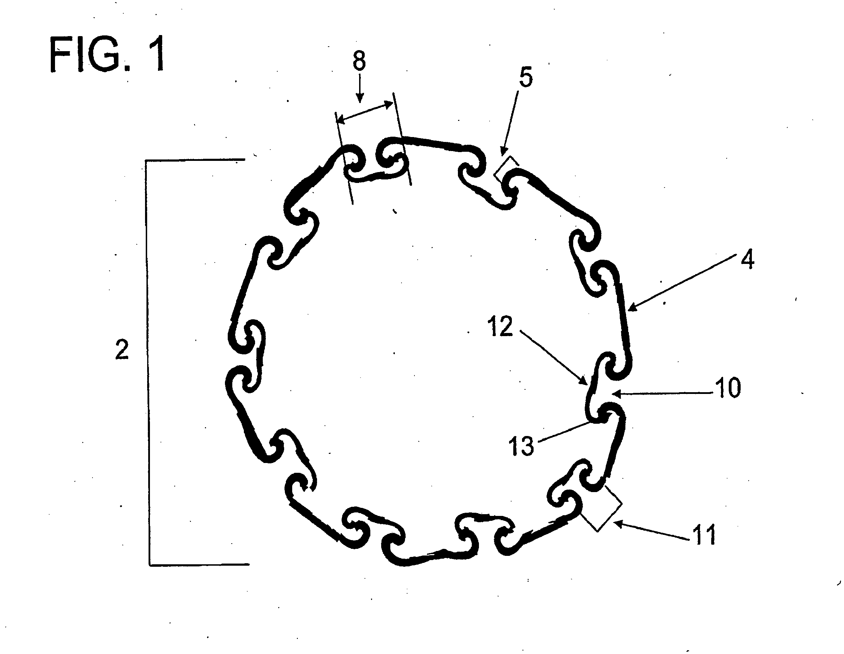

As shown in Figure 1, the catheter balloon 2 is formed of at least one balloon material 4. The balloon material is formed into a balloon having a longitudinal axis and micropleats 8 formed into the balloon. Balloon pleat size and distribution is a function of the inflated diameter, non-inflated diameter and the thickness of the balloon material.

The micropleats 8 are invaginated areas of folded balloon material 4 with at least one fold 5 or bend which forms a pocket 10 of balloon material. The pocket comprises an opening 11 and at least one side of the pocket and a bottom 12 of the pocket 10. The inside of the pocket is a cavity wherein the most distant part of the pocket is adjacent to the bottom of the pocket. The side 13 of the pocket runs from the balloon opening to the bottom of the pocket flanking the opening to the pocket. The opening 11 is ideally located opposite of the bottom 12 of the pocket. The micropleats 8 are distributed about the circumference of the balloon. The bottom of each micropleat pocket is oriented such that it is free of contact or overlap with any other portion of an adjacent micropleat. Each micropleat has at least one fold 5 which allows balloon material to be stored upon itself. While the micropleats are depicted as running longitudinally in the direction of the axis, helical orientations, S-shaped orientations and other configurations of micropleat distribution may be formed. In one example, as shown in Figure 1, the material is folded upon itself to form at least one pocket so that at least 50% of the pocket bottoms are located on the inside of the balloon juxtaposed to the balloon lumen in an uninflated state. In a more preferred example at least 75% of the pocket bottoms are located on the inside of the balloon juxtaposed to the balloon lumen. In a most preferred example, at least 90% of the pocket bottoms are located on the inside of the balloon juxtaposed to the balloon lumen. The catheter balloon has a longitudinal axis with an inner lumen and at least one balloon material forming an outer circumference and at least one micropleat. The micropleat comprises a pocket of balloon material having an opening commencing at the lumen, a bottom and sides where each side folds upon itself to connect the bottom of the pocket to the opening. The micropleat reduces profile and stores the balloon material in a folded configuration until inflation. The micropleats are formed to pull taut in an inflated state. Upon inflation pressure, the balloon exhibits essentially radially symmetric inflation and imparts an equal hydrostatic load during clinical use. Radially symmetric inflation is exhibited when a balloon in an uninflated state can be shown to expand with inflation pressure in a manner allowing points on the balloon to maintain proportionate spacing with each other in the inflated state. When the balloon is used with a stent, the radial inflation symmetry of the balloon also contributes to uniform stent deployment. Such uniform radial inflation has been found to reduce trauma to the vessels into which the balloon is deployed.

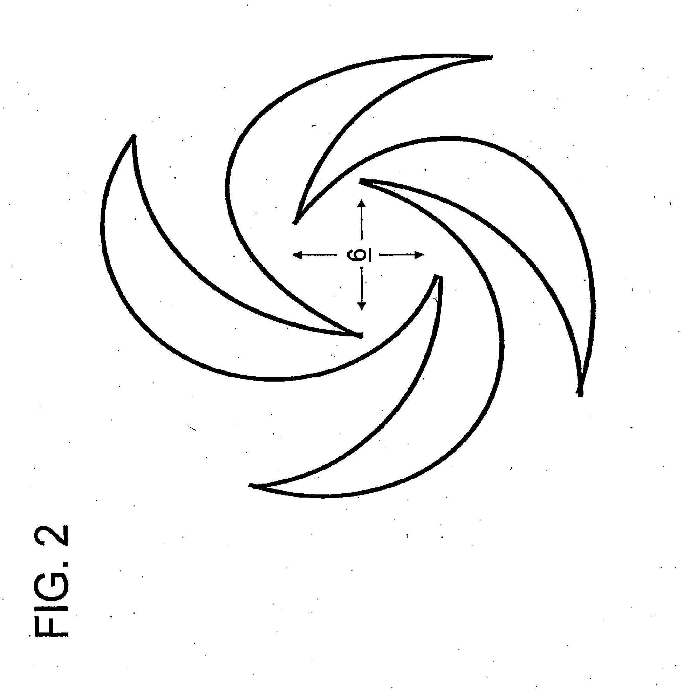

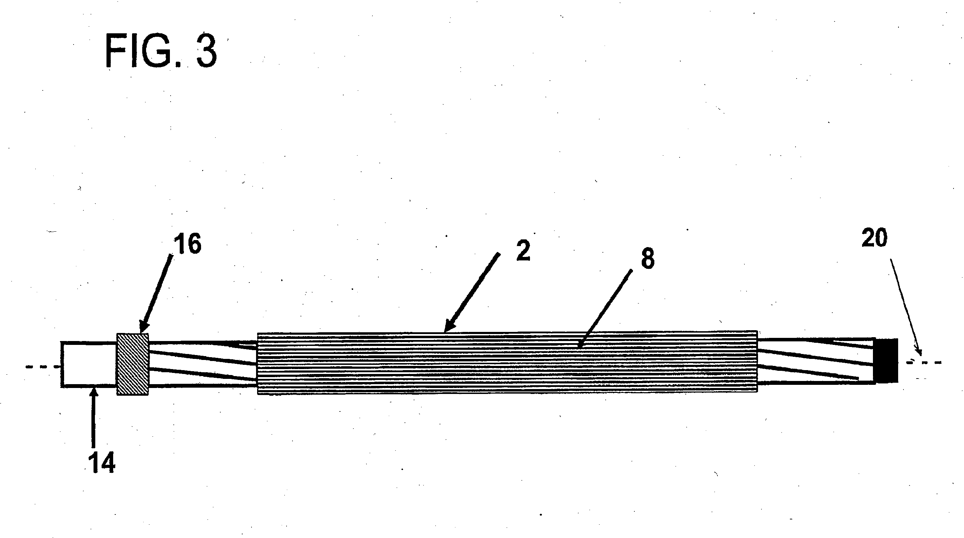

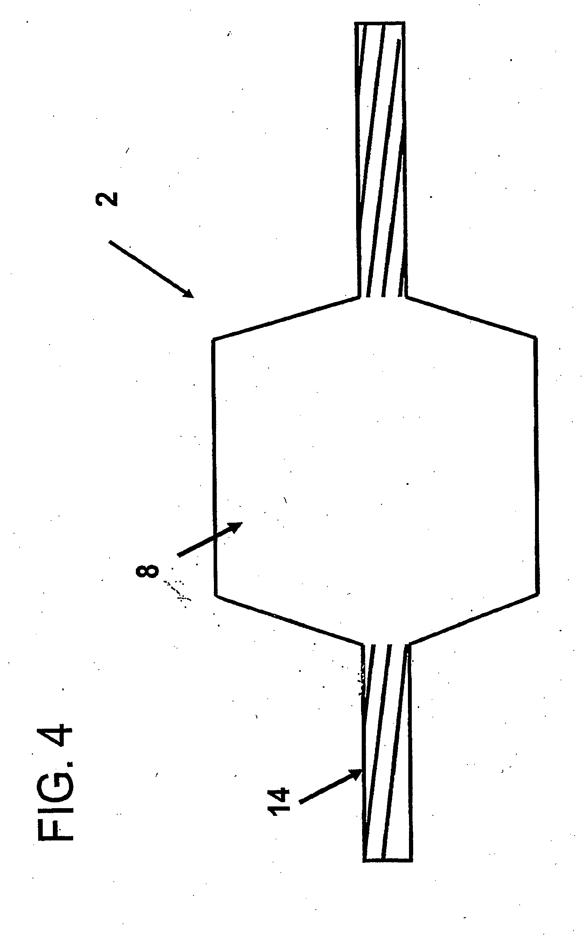

Figure 2 shows a cross section of a traditional folded balloon design, As can be seen, the material is folded upon itself with long folds wrapped around the lumen 6 of the balloon. In this folding pattern, the folds overlap adjacent folds of the balloon around the lumen 6. Internal inflation pressure enters the opening to the pocket and then fills the cavities prior to the pocket sides shortening, thus causing a non-symmetric inflation. As seen in Figure 1, the short walls of the micropleats grow circumferentially upon the addition of an inflation pressure, allowing a symmetrical inflation. The micropleats are symmetric and uniform in their distribution. The micropleats may be oriented in different ways. In one example, as shown in Figure 3, the micropleats 8 are oriented longitudinally, i.e. substantially parallel to the longitudinal axis 20 of the catheter over the working length of the balloon 2. The longitudinal micropleats are evenly distributed about the circumference of the balloon diameter. A balloon seal 16 may be present, distinct from the inflatable balloon portion, if desired, on the catheter shaft 14. The number of micropleats in the balloon may vary, but should be at least six pleats or greater. In a preferred example, the balloon diameter is 1.0 mm or less. However, the micropleats may be utilized on any size balloon. In an inflated state as shown in Figure 4, the micropleats 8 of the balloon pull taut such that they are not visible. The micropleats may be arranged on a formed balloon 2 or on a tubular structure of balloon material.

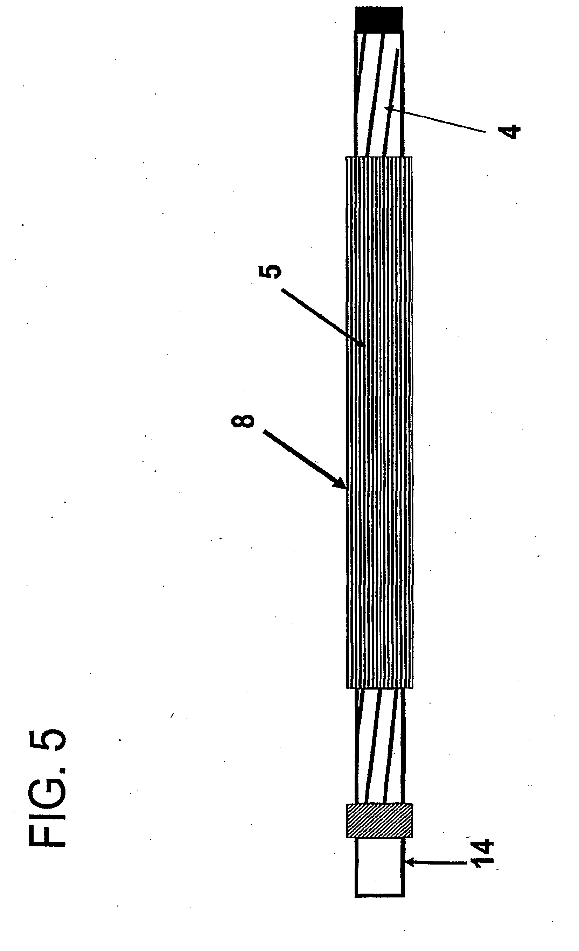

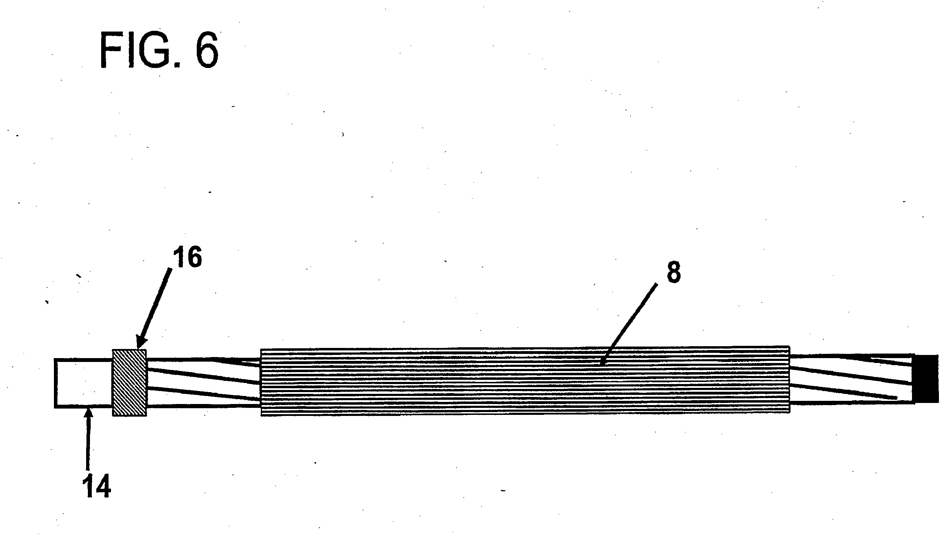

Multiple methods may be used in micropleating a balloon. As shown in Figure 5, an uninflated balloon may be adhered to a catheter shaft 14 and then inflated to achieve an inflated outer diameter. A compressive radial force is then applied to the inflated outer diameter of the balloon to cause deflation of the balloon and create longitudinal folds in the balloon material 4. To set the folds 5 of the micropleats 8 in place, heat, along with a compressive force, is applied so that the balloon maintains its micropleated structure (see Figure 6). In general, the number of pleats generally increases as the ratio of inflated balloon diameter to deflated/packed balloon diameter increases. A balloon seal 16 is shown for reference. An increase in pleats is also observed as the thickness of the balloon wall decreases.

In one aspect, the average micropleat width is less than 1 mm, preferably less than 0.7 mm, independent of inflated/diameter balloon diameters.

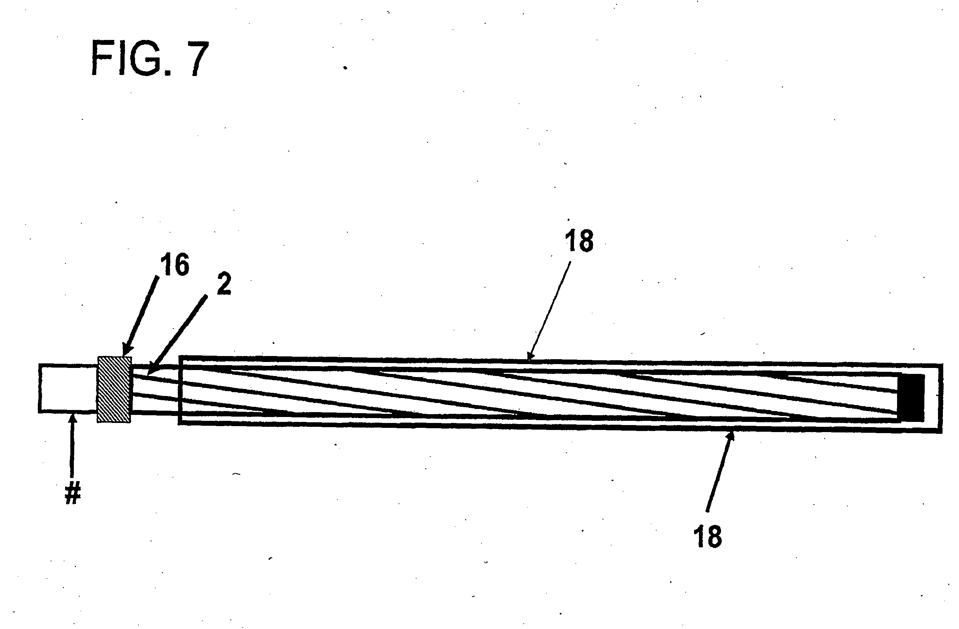

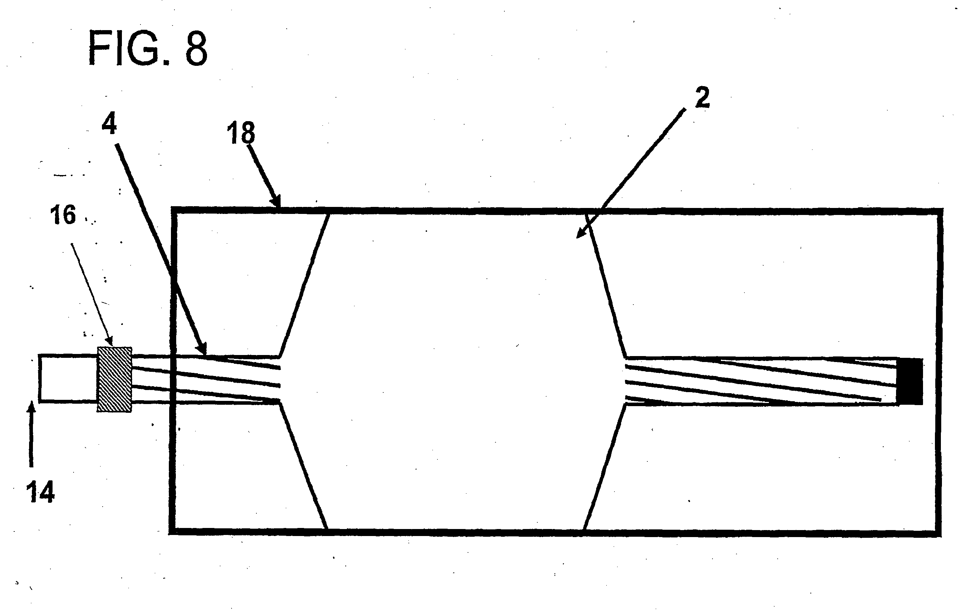

A balloon may be micropleated by positioning the balloon to be packed or micropleated on a catheter shaft 14. Then, the balloon is heat set to the catheter shaft and a small diameter elastomeric tube 18, such as a silicone tube or other suitable material, is rolled over it (see Figure 7). The elastomeric tube is sealed via a balloon seal 16 at one end and inflated on the open end. The tube is inflated to an internal diameter which is greater than the desired outer diameter of the balloon when it is in final inflated form (see Figure 8). The heat set balloon is then inflated inside of the elastomeric tube 18. The elastomeric tube 18 is then deflated onto the inflated heat set balloon. The elastomer in the elastomeric tube 18 in this stage applies a compressive radial force to the inflated balloon causing the balloon to deflate and creating micropleats in the balloon material 4. The elastomeric tube 18 is then removed from the heat set micropleated balloon.

Of the types of balloon material suitable, a material comprising a fluoropolymer and a second polymer is preferred. A common fluoropolymer appropriate for the fluoropolymer component is PTFE or expanded PTFE (ePTFE); however other fibrous reinforcing, highly-oriented materials such as polyolefins, polyesters, polyamides may be used. Polymers suitable for use as second polymers include but are not limited to elastomers such as urethanes, aromatic and aliphatic polyurethanes, thermoplastic polyurethanes, polyester thermoplastics, styrene block copolymers, and silicones. Alternatively, a second fluoropolymer, such as PTFE, may be used for the second polymer. The above described methods enable the balloon profiles of the uninflated balloons to return to essentially the same profile after inflation and subsequent deflation. Essentially, the same profile means that the size of the profile does not change by more than (30%) after deflation as compared to its profile prior to inflation.

One composite film described comprises a porous reinforcing layer and a continuous polymer layer. The porous reinforcing polymer layer is preferably a thin, strong porous membrane that can be made in sheet form. The porous reinforcing polymer can be selected from a group of polymers including, but not limited to, olefin, PEEK, polyamide, polyurethane, polyester, polyethylene, and polytetrafluoroethylene. In a preferred embodiment, the porous reinforcing polymer is expanded polytetrafluoroethylene (ePTFE) may be made in accordance with the teachings of US Patent No. 5,476,589 or US Patent Application No. 11/334,243 to Bacino. In this preferred example, the ePTFE membrane is anisotropic such that it is highly oriented in the one direction. An ePTFE membrane with a matrix tensile value in one direction of greater than 690 megapascals is preferred, and greater than 960 megapascals is even more preferred, and greater than 1,200 megapascals is most preferred. The exceptionally high matrix tensile value of ePTFE membrane allows the composite material to withstand very high hoop stress in the inflated balloon configuration. In addition, the high matrix tensile value of the ePTFE membrane makes it possible for example very thin layers to be used which reduces the deflated balloon profile. A small profile is necessary for the balloon to be able to be positioned in small arteries or veins or orifices. In order for balloons to be positioned in some areas of the body, the balloon catheter must be able to move through a small bend radius, and a thinner walled tube is typically much more supple and capable of bending in this manner without creasing or causing damage to the wall of the vessel.

In another preferred example, the ePTFE membrane is relatively mechanically homogeneous. The mechanically balanced ePTFE membrane can increase the maximum hoop stress that the composite film made therefrom can withstand.

The continuous polymer layer is coated onto at least one side of the porous reinforcing polymer. The continuous polymer layer is preferably an elastomer, such as, but not limited to, aromatic and aliphatic polyurethanes including copolymers, styrene block copolymers, silicones, preferably thermoplastic silicones, fluoro-silicones, fluoroelastomers, THV and latex. In one example, the continuous polymer layer is coated onto only one side of the porous reinforcing polymer. The continuous polymer layer is coated onto both sides of the porous reinforcing polymer. In a preferred example, the continuous polymer layer is imbibed into the porous reinforcing polymer and the imbibed polymer fills the pores of the porous reinforcing polymer. The continuous polymer layer can be applied to the porous reinforcing polymer through any number of conventional methods including, but not limited to, lamination, transfer roll coating, wire-wound bar coating, reverse roll coating, and solution coating or solution imbibing. In a preferred example, the continuous polymer layer is solution imbibed into the porous reinforcing polymer In this example, the continuous polymer layer is dissolved in a suitable solvent and coated onto and throughout the porous reinforcing polymer using a wire-wound rod process. The coated porous reinforcing polymer is then passed through a solvent oven and the solvent is removed leaving a continuous polymer layer coated onto and throughout the porous reinforcing polymer. In some cases, such as when silicone is used as the continuous polymer layer, the coated porous reinforcing polymer may not require the removal of solvent. In another example, the continuous polymer layer is coated onto at least one side of the porous reinforcing polymer and maintained in a "green" state where it can be subsequently cured. For example, an ultraviolet light (UV) curable urethane may be used as the continuous polymer layer and coated onto the porous reinforcing polymer. The composite film comprising the porous reinforcing polymer and the UV curable urethane continuous polymer layer can then be wrapped to form at least one layer of the balloon and subsequently exposed to UV light and cured. A ply is a number of layers applied in a wrapping event. A layer is a single layer of composite film wrapped around the balloon.

While particular embodiments of the present invention have been illustrated and described herein, the present invention should not be limited to such illustrations and descriptions. It should be apparent that changes and modifications may be incorporated and embodied as part of the present invention within the scope of the following claims.

The following examples are intended to be illustrative . These examples are in no way intended to limit the present invention..

### EXAMPLES

### Example 1 - Balloon Example

The balloon portion of a balloon catheter comprising micropleats distributed about the circumference of the balloon was made by inflating a 50 mm section of platinum cured silicone tubing (part # 30400, Saint-Gobain Performance Plastics, Taunton, MA) using a Balloon Development Station Model 210A (Beahm Designs, Campbell, CA) and a dispensing tip (part #: 5121-1-B, EFD, Inc., East Providence, RI). One end of the tube was attached to the dispensing tip with a touhy borst (part #: 80369, Qosina, Edgewood, NY). The silicone tube was inflated to approximately 275 kPa. Once fully inflated, the pressure was quickly lowered to about 130 kPa keeping the tube inflated. While the silicone tube was still inflated, a 1.5 mm PTFE covered mandrel (New England Precision Grinding, Inc., Holliston, MA) was inserted into the tube approximately 100 mm. The tube was deflated and enough hand tension was applied to keep the length constant and under strain while it deflated and compressed onto the mandrel. The tube was cut rolled onto itself about 100 mm in length such that it formed a toroid and removed from the dispensing tip.

The wrapped balloon of this example was comprised of balloon material laid in longitudinal passes about the lumen of the balloon. A longitudinal pass is comprised of one or more layers of material which are laid at similar angles in relation to the longitudinal axis of the balloon. A longitudinal pass comprises a distinctive layer or series of layers of material which are wound or wrapped to form a region or area distinct from surrounding or adjoining parts. It is important to note that a pass may span the entire length of the balloon or in certain instances, such as non-distending regions, the pass may span only a partial length of the balloon.

A layer is considered to be one strand, strip or thickness of balloon material which may be wrapped, folded, laid or weaved over, around, beside or under another strand, strip or thickness of balloon material.

While it is clear that a longitudinal pass may span the entire length of the balloon at a single wrap angle, a longitudinal pass may also comprise a wrapping event in which the wrapping angles may be changed during the continuous longitudinal wrapping, so that in this type of wrapping pattern a single pass may include two or more wrap angles.

The 4 mm diameter x 40 mm long balloon was mounted to a 0.36 mm diameter stainless steel hypotube (Creganna Medical Devices, Parkmore West Galway, Ireland) that had been helically wrapped with approximately three layers of an ePTFE/eFEP film composite as described. The balloon was attached and sealed to the catheter shaft by wrapping an approximately 5 mm wide ePTFE/eFEP film circumferentially around the balloon approximately five times. One band was wrapped on each end of the balloon and was centered over the end of the balloon and the catheter such that it made a seal by contacting both the hypotube shaft and the balloon.

The rolled silicone tube, with an approximately 20 mm long unrolled tail, was removed from the mandrel and then unrolled onto the fully deflated balloon mounted to the catheter shaft described above. The silicone tube was unrolled over the mounted composite balloon such that the end of the unrolled silicone tube protruded just beyond the end of the composite balloon and made a seal on the catheter shaft. The silicone tube was inflated through the tail using the dispensing tip and the Balloon Development Station Model 21 OA to about 130 kPa such that its inner diameter was approximately 4.5 mm. Once the silicone tube was fully inflated, the balloon catheter was inflated to 4 mm diameter using approximately 130 kPa using a separate Balloon Development Station Model 210A and a touhy borst.

The silicone tube was then fully deflated ensuring that there was no change in axial length of the tube. With the axial length of the silicone tube fixed, the balloon was deflated such that the silicone tube applied both a normal compressive force and a tangential shear force against the composite balloon. This shear force was exerted from the decreasing circumference of the inner diameter of the silicone tube and the surface friction between the two surfaces. These combined forces formed a multiplicity of evenly distributed longitudinal micropleats around the circumference of the balloon.

After disconnecting both the balloon catheter and the silicone tube from the inflation devices, the assembly was swaged, or radial compressed, using a heated swaging machine set to 550 kPa and 150°C for 30 seconds. The entire length of the balloon was swaged to heat set the micropleats. The silicone tube was then removed by inflating it to 130 kPa air pressure and unrolling it off of the balloon catheter. This process produced a balloon with tightly packed longitudinal micropleats attached and sealed to a catheter.

### Example 2 - Expansion of Packed Balloon with Micropleats

A 4 mm balloon was folded in accordance with Example 1 above. The balloon was marked in an uninflated state with two parallel rows, each row having 11 linear marked points. The marked points on the first row were directly above and in line with corresponding marked points on the second row, so that the points lined up to form eleven columns. Upon inflation pressure, the marked points in each row radially expanded with the balloon wall and showed a relative equidistant spacing between adjacent marked points. Thus, the rows on the inflated balloon maintained the linear marked points in rows of analogous orientation to the initial marked rows on the uninflated balloon. Additionally, the second row of linear marked points maintained linear rows of analogous orientation to the initial marked rows, and equidistant vertical spacing between the two parallel rows was observed analogous to the initial orientation of the marked points on the uninflated balloon. It was observed that the balloon inflated with radial concentric symmetry.

### Example 3 - Expansion of Packed Balloon with Micropleats

A similar set of markings was conducted on a balloon described in Example 2, with four rows of 11 marked points each oriented to form parallel rows and columns. Upon inflation pressure, the marked points in each row radially expanded with the balloon wall and showed a relative equidistant spacing between adjacent marked points in the corresponding row. Similarly, the columns, radially expanded with the balloon wall and showed a relative equidistant spacing between adjacent marked points in the corresponding columns, and maintained a parallel orientation between the columns and the rows. Thus, both the rows and columns of the inflated balloon maintained the linear marked points in analogous orientation to the initial marked rows on the uninflated balloon, again demonstrating radial symmetry upon inflation.

## Claims

1. A method of micropleating a balloon (2) comprising:
a. heat setting the balloon (2);
b. rolling a small diameter elastomeric tube (18) over the heat set balloon (2);
c. sealing the elastomeric tube (18) on one end;
d. inflating the elastomeric tube (18) on the open end;
e. achieving inflation of the tube (18) to an internal diameter which is greater than the desired outer diameter of the inflated heat set balloon (2);
f. inflating the heat set balloon (2);
g. deflating the elastomeric tube (18);
h. deflating the heat set balloon (2) thus creating axial folds;
i. applying heat and compressive force to set the axial folds, wherein said folds define micropleats (8), where each micropleat (8) comprises a pocket (10) of balloon material (4) having an opening (11) commencing at the outer circumference of the balloon (2), a bottom (12) adjacent the lumen and two sides (13) where each side (13) folds (5) upon itself towards the bottom (12) of the pocket (10) to position the opening (11) of the pocket (10) adjacent the bottom (12) of the pocket (10) and
j. removing the elastomeric tube (18) from over the heat set micropleated balloon (2).

2. The method of claim 1, wherein the elastomeric tube (18) is silicone.

3. The method of claim 1, wherein the balloon (2) is comprised of a fluoropolymer and a polymer.

4. The method of claim 3, wherein the polymer is selected from one or more of the following groups:
an elastomer;
a urethane;
a second fluoropolymer.

5. The method of claim 4, wherein the fluoropolymer is PTFE.

6. The method of any one of claims 1 to 5, wherein:
the folds of step (i) are essentially S-shaped; and/or
the balloon (2) profile in the uninflated state is essentially the same as the balloon (2) profile in a deflated state.

## Patentansprüche

1. Verfahren zum Mikroplissieren eines Ballons (2), umfassend:
a. Thermofixieren des Ballons (2);
b. Rollen einer Elastomerröhre kleinen Durchmessers (18) über den thermofixierten Ballon (2);
c. Abdichten der Elastomerröhre (18) an einem Ende;
d. Aufpumpen der Elastomerröhre (18) am offenen Ende;
e. Vollbringen des Aufpumpens der Röhre (18) auf einen Innendurchmesser, der größer als der gewünschte Außendurchmesser des aufgeblasenen thermofixierten Ballons (2) ist;
f. Aufpumpen des thermofixierten Ballons (2);
g. Gasablassen aus der Elastomerröhre (18);
h. Gasablassen aus dem thermofixierten Ballon (2), wodurch axiale Falten erzeugt werden;
i. Anwenden von Wärme und Druckkraft, um die axialen Falten zu fixieren, worin die Falten Mikroplisseefalten (8) definieren, wobei jede Mikroplisseefalte (8) eine Tasche (10) aus Ballonmaterial (4) umfasst, die Folgendes hat: eine am äußeren Umgang des Ballons (2) beginnende Öffnung (11), einen an das Lumen angrenzenden Boden (12) und zwei Seiten (13), wobei sich jede Seite (13) über sich selbst zum Boden (12) der Tasche (10) hin faltet (5), um die Öffnung (11) der Tasche (10) angrenzend an den Boden (12) der Tasche (10) zu positionieren; und
j. Entfernen der Elastomerröhre (18) vom thermofixierten mikroplissierten Ballon (2).

2. Verfahren nach Anspruch 1, worin die Elastomerröhre (18) aus Silikon ist.

3. Verfahren nach Anspruch 1, worin der Ballon (2) aus einem Fluorpolymer und einem Polymer besteht.

4. Verfahren nach Anspruch 3, worin das Polymer aus einer oder mehreren der folgenden Gruppen ausgewählt wird:
ein Elastomer;
ein Urethan;
ein zweites Fluorpolymer.

5. Verfahren nach Anspruch 4, worin das Fluorpolymer PTFE ist.

6. Verfahren nach einem der Ansprüche 1 bis 5, worin:
die Falten von Schritt (i) im Wesentlichen s-förmig sind; und/oder das Profil des Ballons (2) im nicht aufgepumpten Zustand im Wesentlichen das gleiche wie das Profil des Ballons (2) in einem Zustand nach dem Gasablassen ist.

## Revendications

1. Procédé de microplissage d'un ballon (2) comprenant les étapes consistant à :
a. thermofixer le ballon (2) ;
b. rouler un tube élastomère de faible diamètre (18) sur le ballon thermofixé (2);
c. fermer de manière étanche le tube élastomère (18) à une extrémité ;
d. gonfler le tube élastomère (18) à l'extrémité ouverte ;
e. procéder au gonflage du tube (18) jusqu'à un diamètre interne qui est supérieur au diamètre externe souhaité du ballon thermofixé gonflé (2) ;
f. gonfler le ballon thermofixé (2) ;
g. dégonfler le tube élastomère (18) ;
h. dégonfler le ballon thermofixé (2), ce qui crée des plis axiaux ;
i. appliquer de la chaleur et une force de compression pour fixer les plis axiaux, dans lequel lesdits plis définissent des microplis (8), chaque micropli (8) comprenant une poche (10) de matériau de ballon (4) présentant une ouverture (11) commençant à la circonférence extérieure du ballon (2), un fond (12) adjacent à la lumière et aux deux côtés (13), chaque côté (13) se repliant (5) sur lui-même en direction du fond (12) de la poche (10) afin de positionner l'ouverture (11) de la poche (10) de manière adjacente au fond (12) de la poche (10) et
j. retirer le tube élastomère (18) de dessus le ballon microplissé thermofixé (2).

2. Procédé selon la revendication 1, dans lequel le tube élastomère (18) est en silicone.

3. Procédé selon la revendication 1, dans lequel le ballon (2) est constitué d'un fluoropolymère et d'un polymère.

4. Procédé selon la revendication 3, dans lequel le polymère est sélectionné parmi un ou plusieurs des groupes suivants :
un élastomère ;
un uréthane ;
un second fluoropolymère.

5. Procédé selon la revendication 4, dans lequel le fluoropolymère est du PTFE.

6. Procédé selon l'une quelconque des revendications 1 à 5, dans lequel :
les plis de l'étape (i) sont essentiellement en forme de S ; et/ou
le profil de ballon (2) dans l'état non gonflé est essentiellement le même que le profil de ballon (2) dans un état dégonflé.
